(19) Europäisches Patentamt
European Patent Office
Office européen des brevets

(11) **EP 0 755 673 B1**

(12) **FASCICULE DE BREVET EUROPEEN**

(45) Date de publication et mention
de la délivrance du brevet:
**17.03.1999 Bulletin 1999/11**

(51) Int. Cl.⁶: **A61K 7/48**

(21) Numéro de dépôt: **96401431.0**

(22) Date de dépôt: **27.06.1996**

(54) **Composition stable contenant un actif cosmétique et/ou dermatologique sensible à l'eau**

Stabile Zusammensetzung, die einen wasserempfindlichen kosmetischen und/oder dermatologischen Wirkstoff enthält

Stable composition containing a water-sensitive cosmetic and/or dermatalogic agent

(84) Etats contractants désignés:
**DE ES FR GB IT**

(30) Priorité: **25.07.1995 FR 9509029**

(43) Date de publication de la demande:
**29.01.1997 Bulletin 1997/05**

(73) Titulaire: **L'OREAL**
**75008 Paris (FR)**

(72) Inventeurs:
• **Afriat, Isabelle**
  **75014 Paris (FR)**
• **Gagnebien, Didier**
  **92320 Chatillon (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
EP-A- 0 755 672     FR-A- 1 397 399
LU-A- 61 123

• CHEMICAL ABSTRACTS, vol. 120, no. 26, 27 Juin 1994 Columbus, Ohio, US; abstract no. 330796h, TAKUJI MASUNAGA ET AL: "The protease as a cleansing agent and its stabilization by chemical modification" page 484; XP002016491 & J. SCCJ, vol. 27, no. 3, 1993, TOKYO, pages 276-288,
• CHEMICAL ABSTRACTS, vol. 112, no. 14, 2 Avril 1990 Columbus, Ohio, US; abstract no. 124943k, page 398; XP002016492 & JP-A-01 238 510 (TAKARA SHUZO CO., LTD) 22 Septembre 1989
• PATENT ABSTRACTS OF JAPAN vol. 13, no. 515 (C-655) [3863] , 17 Novembre 1989 & JP-A-01 207220 (LION CORP), 21 Août 1989,

## Description

La présente invention se rapporte à une composition à application topique contenant un actif stabilisé, utilisable en particulier dans les domaines cosmétique et/ou dermatologique pour nettoyer et/ou soigner et/ou protéger la peau et/ou les fibres kératiniques.

Il est connu d'introduire dans les compositions cosmétiques et/ou dermatologiques des actifs en vue d'apporter des traitements spécifiques à la peau et/ou aux cheveux, par exemple pour nettoyer la peau, pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire, pour traiter la séborrhée des cheveux.

Par exemple, il est connu d'introduire dans les compositions cosmétiques des enzymes, et notamment des protéases utilisées pour leurs propriétés protéolytiques. Ces enzymes sont recherchées dans le domaine cosmétique pour leur pouvoir lissant et nettoyant, et leur aptitude à éliminer les cellules mortes de la peau.

Malheureusement, certains actifs, et en particulier ceux cités ci-dessus, présentent l'inconvénient d'être instables en milieu aqueux et d'être facilement dégradés ou modifiés sous l'influence de l'eau. Ils perdent ainsi rapidement de leur activité au cours du temps et cette instabilité va à l'encontre de l'efficacité recherchée.

Aussi, différents moyens ont été envisagés pour pallier à cet inconvénient. En particulier, il a été envisagé de mettre un actif, notamment une enzyme dans une composition pulvérulente (voir le document JP-A-63-130514). D'ailleurs, la plupart des produits de nettoyage de la peau contenant une enzyme se présentent sous cette forme. Il a été aussi envisagé d'utiliser ces actifs, et notamment les enzymes, sous forme immobilisée sur des supports polymériques (voir le document JP-A-61-207499) ou dans des microcapsules (voir le document JP-A-61-254244). Malheureusement, certains de ces moyens nécessitent une mise en oeuvre particulière, ce qui accroît le coût et le temps de préparation de la composition.

Une autre solution consiste à les incorporer dans un milieu liquide anhydre (voir le document US-A-5322683). Malheureusement, cette solution limite la forme galénique de la composition et ne permet pas l'incorporation d'actifs hydrophiles.

Il subsiste donc le besoin d'une composition pour application topique contenant des actifs cosmétiques et/ou dermatologiques sensibles à l'eau, dans laquelle ces derniers conservent toutes leurs propriétés et donc leur efficacité au cours du temps.

La demanderesse a maintenant trouvé de manière inattendue que l'utilisation d'au moins un polyol liant l'eau dans une composition topique contenant un actif sensible à l'eau, en quantité efficace pour obtenir une valeur d'activité en eau de la composition, inférieure ou égale à 0,85, et d'au moins un agent structurant permet d'éviter la dégradation de l'actif.

Aussi, la présente invention a pour objet une composition stable à application topique contenant au moins un actif à action topique sensible à l'eau et au moins un polyol, caractérisée en ce qu'elle est exempte de sel de calcium, en ce que le polyol est présent en une quantité efficace pour obtenir une valeur d'activité en eau de la composition, inférieure ou égale à 0,85 et au moins un homopolymère acrylique ou méthacrylique, complexant l'eau et le(s) polyol(s), et/ou une huile.

L'invention se rapporte aussi à une composition stable à application topique contenant au moins un actif à action topique sensible à l'eau et au moins un polyol, caractérisée en ce que le polyol est présent en une quantité allant de 40 à 99,99 % en poids par rapport au poids total de la composition et en ce qu'elle comprend au moins un homopolymère acrylique ou méthacrylique, complexant l'eau et le(s) polyol(s), et/ou une huile.

Certes, il est connu que la teneur en eau peut avoir une influence sur la stabilité des actifs sensibles à l'eau, mais il n'a jamais été décrit ni suggéré que la présence de polyol et d'un agent structurant puisse éviter la dégradation de tels actifs. Ainsi, le document de D. Tzanos (Behavior of enzymes by controlling the medium water activity ; Riv. Ital. Essenze, Profumi, Piante Off., Aromi, Saponi, Cosmet., Aerosol, 1977, vol.59, n°5, pages 208-211) incite l'homme du métier à utiliser des tensioactifs pour la stabilisation des enzymes en milieu aqueux ou à fixer les enzymes sur un support poreux. En revanche, il écarte l'homme du métier d'utiliser des glycols.

Par ailleurs, le document US-A-5356800 décrit un procédé de stabilisation des enzymes consistant à utiliser un mélange comprenant un alcool ou un glycol, une alkyldiamine oxyéthylénée et un oxyde d'amine. Selon ce document, la stabilisation des enzymes ne peut être obtenue qu'en utilisant le mélange décrit.

En outre, le document JP-A-01-283213 décrit une composition de nettoyage contenant une enzyme et un polyol. Selon ce document, l'activité enzymatique est stabilisée par addition d'une protéine telle que le collagène, l'élastine ou l'albumine.

Le document FR-A-1397399 décrit un procédé pour stabiliser les protéases consistant à utiliser un mélange de polyol et de sel de calcium. Selon ce document, la présence de sel de calcium est indispensable à la stabilisation de la protéase.

Par ailleurs, il est connu du document J. Soc. Cosm. Chem. Jap., 1993, 27(3), p.276-288 que l'on peut stabiliser les protéases en les modifiant chimiquement et que l'addition de polyols contribue à améliorer la stabilité de la protéase modifiée. Selon ce document, la modification chimique est nécessaire pour obtenir la stabilisation des enzymes.

Or, il a été maintenant trouvé que, dans le cas des compositions topiques, les polyols utilisés en une quantité suffisante et en association avec un agent structurant peuvent empêcher la dégradation des actifs sensibles à l'eau.

Ainsi, la présente invention a aussi pour objet l'utilisation, dans une composition à application topique exempte de sel de calcium et contenant au moins un actif à action topique sensible à l'eau, d'au moins un polyol en une quantité efficace pour obtenir une valeur d'activité en eau de la composition, inférieure ou égale à 0,85, et d'au moins un homopolymère acrylique ou méthacrylique, complexant l'eau et le(s) polyol(s), et/ou une huile, en vue de stabiliser l'actif sensible à l'eau.

De préférence, le polymère est choisi parmi les polymères acryliques et méthacryliques.

De préférence, la quantité du ou des polyols doit être telle que la valeur d'activité en eau de la composition est inférieure ou égale à 0,7.

L'activité en eau $a_w$ d'un milieu contenant de l'eau est le rapport de la pression de vapeur d'eau du produit 《 $P_{H2O}$ produit 》 et de la pression de vapeur de l'eau pure 《 $P_{H2O}$ pur 》 à la même température. Elle peut être exprimée aussi comme le rapport du nombre de molécules d'eau 《 $N_{H2O}$ 》 sur le nombre de molécules totales 《 $N_{H2O} + N_{corps\ dissous}$ 》, qui tient compte de celles des corps dissous 《 $N_{corps\ dissous}$ 》.

Elle est donnée par les formules suivantes :

$$a_w = \frac{P_{H2O}\ produit}{P_{H2O}\ pur} = \frac{N_{H2O}}{N_{H2O} + N_{corps\ dissous}}$$

On peut utiliser différentes méthodes pour mesurer l'activité en eau. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable de l'activité en eau.

Le polyol utilisé selon l'invention peut être notamment choisi parmi la glycérine et les glycols, en particulier le propylène glycol et les polyéthylène glycols.

La quantité de polyol(s) à utiliser dans la composition de l'invention dépend du type de composition (gel ou émulsion) et des autres constituants présents dans la composition. Cette quantité doit être suffisante pour atteindre la valeur recherchée d'activité en eau. Le ou les polyols utilisés selon l'invention sont de préférence présents en une quantité d'au moins 40 % en poids, de préférence allant de 40 à 99,99 % en poids, et mieux de 60 à 80 % en poids par rapport au poids total de la composition.

Selon un mode de réalisation préféré de l'invention, le ou les polyols se trouvent totalement ou partiellement sous forme complexée avec un polymère acrylique ou méthacrylique. Le polymère peut également comprendre de l'eau liée, c'est-à-dire être complexé avec un mélange d'eau et de polyol(s).

On entend par polymère acrylique ou méthacrylique un homopolymère ou un copolymère d'acide acrylique ou méthacrylique ou un homopolymère ou un copolymère d'un dérivé d'acide acrylique ou méthacrylique.

La quantité de polymères avec le ou les polyols et éventuellement l'eau liée, dans la composition selon l'invention va de préférence de 70 à 99,99 % en poids, et mieux de 80 à 95 % en poids par rapport au poids total de la composition.

On peut citer comme homopolymère complexant l'eau et les polyols, ceux vendus sous les dénominations de Norgel et de Lubrajel CG par la société Guardian. Ces polymères sont des polyacrylates de glycéryle complexés avec plus de 65 % de glycérine et/ou de propylène glycol et moins de 35 % en poids d'eau. Ces polymères apportent le polyol et l'eau complexés, et éventuellement jouent en outre le rôle de gélifiant de la composition.

Les tests comparatifs présentés ci-dessous montrent que seules les compositions ayant une valeur d'activité en eau au plus égale à 0,85 permettent une bonne conservation de l'activité des actifs topiques sensibles à l'eau, et notamment une bonne conservation de l'activité enzymatique des enzymes.

Les actifs sensibles à l'eau qui peuvent être utilisés selon l'invention sont notamment les enzymes (par exemple lactoperoxydase, lipase, protéase, phospholipase, cellulases), les extraits naturels tels que thé vert, extrait de mélisse, extrait de thym, les oligomères procyannidoliques (OPC) tels que l'OPC d'aubépine, l'OPC de pin et l'OPC de raisin, les vitamines et notamment l'acide ascorbique (vitamine C) et ses esters, le rétinol (vitamine A) et ses esters, les dérivés phosphatés et glucosilés, l'urée et la rutine.

Le ou les actifs sensibles à l'eau utilisés sont de manière avantageuse une enzyme, et plus particulièrement une protéase. Cette protéase peut être choisie par exemple parmi celle vendue sous la dénomination commerciale "Subtilisine SP 544" par la société Novo Nordisk et celle vendue sous la dénomination commerciale "Lysoveg" par la société Laboratoires Sérobiologiques de Nancy.

La quantité d'actif sensible à l'eau dans la composition selon l'invention dépend du type d'actif utilisé. De manière générale, le ou les actifs peuvent être utilisés dans la composition selon l'invention en une quantité allant de 0,001 à 15 % en poids, de préférence de 0,01 à 10 %, et mieux de 0,05 à 5 % en poids par rapport au poids total de la composition.

Comme huiles utilisables dans l'invention, on peut citer les huiles minérales (huile de vaseline), les huiles végétales (huile de jojoba), les huiles animales, les huiles de synthèse (oléate de décyle), les huiles siliconées (cyclométhicone, polydiméthylsiloxane, diméthicone) et les huiles fluorées (perfluoropolyéthers). La ou les huiles peuvent être présentes en une quantité allant de 5 à 60 %, et de préférence de 5 à 40 % en poids par rapport au poids total de la composition.

En outre, la composition selon l'invention peut contenir un ou plusieurs sels dont la présence va encore améliorer la stabilité de l'actif qu'elle contient. Comme sels, on peut citer en particulier les sels de magnésium et les sels de sodium, et plus spécialement le sulfate de magnésium, le chlorure de magnésium et le chlorure de sodium. Le ou les sels peuvent être présents en une quantité allant de 0,1 à 30 % et de préférence de 2 à 12 % en poids par rapport au poids total de la composition.

La composition selon l'invention contient un milieu topiquement acceptable, c'est-à-dire compatible avec la peau et les cheveux, et constitue notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

Aussi, l'invention a encore pour objet l'utilisation de la composition selon l'invention pour nettoyer et/ou protéger la peau et/ou les fibres kératiniques, c'est-à-dire les cheveux et/ou les cils.

La présente invention a aussi pour objet une composition nettoyante pour la peau et/ou les fibres kératiniques contenant au moins un actif nettoyant sensible à l'eau et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un homopolymère acrylique ou méthacrylique, complexant l'eau et le(s) polyol(s), et/ou une huile.

L'invention a enfin pour objet un procédé cosmétique et/ou dermatologique pour nettoyer et/ou protéger la peau et/ou les fibres kératiniques, caractérisé en ce qu'il consiste à appliquer sur la peau et/ou les fibres kératiniques une composition telle que définie ci-dessus.

La composition selon l'invention peut se présenter notamment sous forme d'une solution, d'un gel, d'une émulsion eau-dans-huile ou huile-dans-eau constituant des crèmes, des onguents, des lotions ou des laits. Cette composition peut comprendre aussi des microcapsules, des microparticules, ou des vésicules lipidiques de type ionique et/ou non ionique. Ces différentes formes de composition sont préparées selon les méthodes usuelles.

Ces compositions constituent notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu.

Lorsque la composition de l'invention est une émulsion, la proportion de la phase grasse peut aller de 10 à 80 % en poids, et de préférence de 20 à 40 % en poids par rapport au poids total de la composition. L'émulsion comprend de préférence au moins un agent dispersant choisi parmi les émulsionnants, les vésicules et les particules. Les huiles, les émulsionnants et éventuellement les coémulsionnants utilisés dans la composition sous forme d'émulsion sont choisis parmi ceux classiquement utilisés dans les domaine cosmétique et dermatologique. L'émulsionnant et le coémulsionnant peuvent être présents, dans la composition, en une proportion allant de 1 à 10 % en poids, et de préférence de 2 à 6 % en poids par rapport au poids total de la composition.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles en plus des actifs sensibles à l'eau, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 15 % du poids total de la composition. Ces adjuvants, selon leur nature, peuvent être introduits dans la phase grasse, dans la phase aqueuse et/ou dans les vésicules lipidiques.

La phase grasse peut comprendre en plus des huiles indiquées ci-dessus des matières grasses telles que des alcools gras, des acides gras (acide stéarique), des cires (cire de silicone).

Comme tensioactifs moussants utilisables dans l'invention, on peut citer par exemple le cocoamphodiacétate de disodium (Miranol C2M vendu par la société Rhône-Poulenc) et le décyl éther de glucose à 55 % dans l'eau (Oramix NS10 vendu par la société Seppic). La teneur en eau de ces matières premières fait partie de la quantité totale d'eau dans la composition.

Comme émulsionnants utilisables dans l'invention, on peut citer par exemple les émulsionnants siliconés comme les alkyldiméthicone copolyols tels que le cétyldiméthicone copolyol vendu par la société Goldschmidt sous la dénomination Abil EM-90, ou le mélange de diméthicone copolyol et cyclométhicone, vendu par la société Dow Corning sous la dénomination 3225C Formulation Aid.

Comme actifs hydrophiles, on peut utiliser par exemple les protéines ou les hydrolysats de protéine, les acides aminés, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles.

**Test de stabilité de l'activité enzymatique :**

On a déterminé l'activité enzymatique d'une enzyme contenue dans un gel aqueux selon l'invention et dans deux gels comparatifs en utilisant la méthode à la caséine. Selon cette méthode, la caséine utilisée comme substrat est hydrolysée par l'enzyme en libérant des acides aminés qui sont ensuite quantifiés par colorimétrie à l'aide du réactif de Folin-Ciocalteu. L'absorbance colorimétrique lue est d'autant plus grande que la quantité d'enzyme est importante.

Les gels testés contenaient 1 % p/p de protéase (protéase SP 544) et avaient la composition suivante :

- Gel I (selon l'invention) : 99 % de Norgel (soit 0,99 % de polymère acrylique, 66,3 % de polyol et 30,7 % d'eau) et 1 % de protéase.
- Gel II (comparatif) : 99 % d'alginate de propylène glycol estérifié à 80-85 %, à 0,5 % dans l'eau et 1 % de protéase.
- Gel III (comparatif) : 99 % de polysaccharide (Fucogel 1000 : Biosaccharide gum-1 vendu par la société Solabia à base de fucose, galactose et d'acide galacturonique) et 1 % de protéase.

Le tableau suivant donne les résultats en pourcentage d'activité enzymatique restante après deux mois :

| Gel | Activité en eau du gel $a_w$ | % d'activité enzymatique |
|---|---|---|
| Gel I | 0,65 | 71 % |
| Gel II | 0,989 | 0 % |
| Gel III | 0,967 | 0 % |

Ces résultats montrent que seul le gel I selon l'invention permet la conservation de l'activité enzymatique de la protéase.

Les exemples ci-après de compositions selon l'invention, sont donnés à titre d'illustration et sans caractère limitatif. Les quantités y sont données en % en poids.

**Exemple 1 : Gel**

| Norgel | 85 % |
|---|---|
| Subtilisine SP 544 | 0,1 % |
| Eau | qsp 100 % |

On obtient un gel translucide, utilisable comme gel exfoliant. Son activité en eau est de 0,735 ± 0,05.

Après 2 mois à la température ambiante, l'activité enzymatique de la subtilisine SP 544 est encore de 80 %.

5

**Exemple 2 : Emulsion eau-dans-huile**

| Phase aqueuse : | |
|---|---|
| Norgel | 71,5 % |
| NaCl | 0,5 % |
| Phase huileuse : | |
| Cétyldiméthicone copolyol (Abil EM-90 vendu par la société Goldschmidt) (émulsionnat) | 2 % |
| Huile de jojoba | 4 % |
| Polydiméthylsiloxane | 8 % |
| Huile de vaseline | 10 % |
| Oléate de décyle | 3,9 % |
| Subtilisine SP544 | 0,1 % |

Le mode opératoire pour préparer l'émulsion est le suivant : On prépare la phase aqueuse d'une part et la phase huileuse d'autre part, et on émulsionne la phase aqueuse dans la phase huileuse à température ambiante sous agitation à l'homogénéisateur.

On obtient une crème blanche apte à faciliter l'élimination des cellules de la peau et à éclaircir le teint. Son activité en eau est de $0,62 \pm 0,02$.

Après 2 mois à la température ambiante, l'activité enzymatique de la subtilisine SP 544 est encore de 100 %.

**Exemple 3 : Gel de nettoyage**

| | |
|---|---|
| Subtilisine SP544 | 0,04 % |
| Norgel | 83 % |
| Miranol C2M (vendu par la société Rhône-Poulenc) | 16 % |
| Eau | qsp 100 % |

On obtient un gel de nettoyage moussant pour le visage et le corps, rinçable à l'eau. Son activité en eau est de $0,67 \pm 0,02$.

**Exemple 4 : Gel de nettoyage**

| | |
|---|---|
| Norgel | 88,97 % |
| Lysoveg | 0,03 % |

(suite)

| Oramix NS10 (vendu par la société Seppic) | 11 % |
|---|---|

On obtient un gel de nettoyage moussant pour le visage et le corps, rinçable à l'eau. Son activité en eau est de 0,68 ± 0,02.

**Exemple 5 : Emulsion eau-dans-huile**

| Phase huileuse : | |
|---|---|
| Diméthicone copolyol et cyclométhicone («3225C Formulation Aid» vendu par Dow Corning) | 22,6 % |
| Diméthicone | 4,9 % |
| Huile minérale | 3 % |
| Phase aqueuse : | |
| Glycérine | 45,5 % |
| Sulfate de magnésium (stabilisant) | 2 % |
| Subtilisine SP544 | 0,05 % |
| Propylène glycol | 8 % |
| Eau | qsp 100 % |

L'émulsion est préparée de la même manière que dans l'exemple 2.

On obtient une crème blanche pour le lissage de la peau, dont l'activité en eau est de 0,63 ± 0,02.

Après 2 mois à la température ambiante, l'activité enzymatique de la subtilisine SP 544 est encore de 90 %.

**Exemple 6 : Emulsion eau-dans-huile**

| Phase huileuse : | |
| --- | --- |
| Diméthicone copolyol et cyclométhicone (《3225C | 22,8 % |
| Formulation Aid》vendu par Dow Corning) | |
| Diméthicone et triméthylsiloxysilicate (《593 | 5 % |
| Fluid》vendu par Dow Corning) | |
| Octyl palmitate | 6,7 % |
| Amidon de maïs | 8 % |
| Nylon-12 | 5 % |
| Phase aqueuse : | |
| Glycérine | 8 % |
| Propylène glycol | 8 % |
| Chlorure de magnésium | 6 % |
| Subtilisine SP544 | 0,1 % |
| Eau | qsp 100 % |

L'émulsion est préparée de la même manière que dans l'exemple 2.

On obtient une crème blanche pour le lissage de la peau, dont l'activité en eau est de $0,75 \pm 0,02$.

On pourrait remplacer selon l'invention, dans les exemples ci-dessus, la Subtilisine SP544 par d'autres enzymes, l'acide ascorbique, le thé vert et les autres actifs sensibles à l'eau cités ci-dessus.

## Revendications

1. Composition stable à application topique contenant au moins un actif à action topique sensible à l'eau et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un homopolymère acrylique ou méthacrylique, complexant l'eau et le(s) polyol(s), et/ou une huile.

2. Composition selon la revendication 1, caractérisée en ce que le polyol est présent en une quantité efficace pour obtenir une valeur d'activité en eau de la composition, inférieure ou égale à 0,7.

3. Composition selon l'une quelconque des revendications précédentes, caractérisée en ce que le polyol est présent en une quantité d'au moins 40 % en poids par rapport au poids total de la composition.

4. Composition stable à application topique contenant au moins un actif à action topique sensible à l'eau et au moins un polyol, caractérisée en ce que le polyol est présent en une quantité allant de 40 à 99,99 % en poids par rapport au poids total de la composition et en ce qu'elle comprend au moins un homopolymère acrylique ou méthacrylique, complexant l'eau et le(s) polyol(s), et/ou une huile.

5. Composition selon l'une quelconque des revendications précédentes, caractérisée en ce que le polyol est choisi dans le groupe comprenant la glycérine et les glycols.

6. Composition selon l'une quelconque des revendications précédentes, caractérisée en ce que le polymère, le polyol et l'eau liée sont présents en une quantité allant de 70 à 99,99 % en poids par rapport au poids total de la composition.

**7.** Composition selon l'une quelconque des revendications précédentes, caractérisée en ce que l'huile est présente en une quantité allant de 5 à 60 % en poids par rapport au poids total de la composition.

**8.** Composition selon l'une quelconque des revendications précédentes, caractérisée en ce que l'huile est choisie parmi les huiles minérales, les huiles végétales, les huiles animales, les huiles de synthèse, les huiles siliconées et les huiles fluorées.

**9.** Composition selon l'une quelconque des revendications précédentes, caractérisée en ce que l'actif à action topique sensible à l'eau est choisi dans le groupe comprenant les enzymes, les extraits naturels, les oligomères procyannidoliques, les vitamines, les dérivés phosphatés et glucosilés, l'urée et la rutine.

**10.** Composition selon la revendication précédente, caractérisée en ce que l'actif à action topique sensible à l'eau est choisi dans le groupe comprenant une protéase, le thé vert, l'acide ascorbique, le rétinol et ses esters.

**11.** Composition selon l'une quelconque des revendications précédentes, caractérisée en ce que l'actif sensible à l'eau est présent en une concentration allant de 0,001 à 15 % en poids par rapport au poids total de la composition.

**12.** Composition selon l'une quelconque des revendications précédentes, caractérisée en ce qu'elle comprend en outre au moins un sel choisi parmi les sels de magnésium et de sodium.

**13.** Composition selon l'une quelconque des revendications précédentes, caractérisée en ce qu'elle comprend en outre au moins un adjuvant lipophile ou hydrophile choisi parmi les conservateurs, les antioxydants, les parfums, les charges, les filtres, les séquestrants, les huiles essentielles, les matières colorantes, les actifs hydrophiles ou lipophiles, et les vésicules lipidiques.

**14.** Composition selon l'une quelconque des revendications précédentes, caractérisée en ce qu'elle se présente sous forme d'émulsion et en ce qu'elle comprend, en outre, au moins un agent dispersant choisi parmi les émulsionnants, les vésicules et les particules.

**15.** Composition nettoyante pour la peau et/ou les fibres kératiniques contenant au moins un actif nettoyant sensible à l'eau et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un homopolymère acrylique ou méthacrylique, complexant l'eau et le(s) polyol(s), et/ou une huile.

**16.** Utilisation, dans une composition à application topique exempte de sel de calcium et contenant au moins un actif à action topique sensible à l'eau, d'au moins un polyol en une quantité efficace pour obtenir une valeur d'activité en eau de la composition, inférieure ou égale à 0,85, et d'au moins un homopolymère acrylique ou méthacrylique, complexant l'eau et le(s) polyol(s), et/ou une huile, en vue de stabiliser l'actif sensible à l'eau.

**17.** Procédé cosmétique pour nettoyer et/ou protéger la peau et/ou les fibres kératiniques, caractérisé en ce qu'il consiste à appliquer sur la peau et/ou les fibres kératiniques une composition selon une des revendications 1 à 15.

**Claims**

**1.** Stable composition for topical application containing at least one water-sensitive active agent with a topical action and at least one polyol, characterized in that the said composition contains no calcium salt, in that the polyol is present in a quantity which is effective for obtaining a water activity value of the composition of less than or equal to 0.85, and in that the said composition comprises at least one acrylic or methacrylic homopolymer, complexing the water and the polyol(s), and/or an oil.

**2.** Composition according to Claim 1, characterized in that the polyol is present in a quantity which is effective for obtaining a water activity value of the composition of less than or equal to 0.7.

**3.** Composition according to either one of the preceding claims, characterized in that the polyol is present in a quantity of at least 40 % by weight relative to the total weight of the composition.

**4.** Stable composition for topical application containing at least one water-sensitive active agent with a topical action and at least one polyol, characterized in that the polyol is present in a quantity ranging from 40 to 99.99 % by weight, relative to the total weight of the composition, and in that the said composition comprises at least one acrylic or methacrylic homopolymer, complexing the water and the polyol(s), and/or an oil.

5. Composition according to any one of the preceding claims, characterized in that the polyol is chosen from the group consisting of glycerol and glycols.

6. Composition according to any one of the preceding claims, characterized in that the polymer, the polyol and the bound water are present in a quantity ranging from 70 to 99.99 % by weight, relative to the total weight of the composition.

7. Composition according to any one of the preceding claims, characterized in that the oil is present in a quantity ranging from 5 to 60 % by weight, relative to the total weight of the composition.

8. Composition according to any one of the preceding claims, characterized in that the oil is chosen from mineral oils, vegetable oils, animal oils, synthetic oils, silicone oils and fluorinated oils.

9. Composition according to any one of the preceding claims, characterized in that the water-sensitive active agent with a topical action is chosen from the group consisting of enzymes, natural extracts, procyanidol oligomers, vitamins, phosphated and glucosylated derivatives, urea and rutin.

10. Composition according to the preceding claim, characterized in that the water-sensitive active agent with a topical action is chosen from the group consisting of a protease, green tea, ascorbic acid, retinol and its esters.

11. Composition according to any one of the preceding claims, characterized in that the water-sensitive active agent is present in a concentration ranging from 0.001 to 15 % by weight, relative to the total weight of the composition.

12. Composition according to any one of the preceding claims, characterized in that it additionally comprises at least one salt, chosen from magnesium salt and sodium salt.

13. Composition according to any one of the preceding claims, characterized in that it additionally comprises at least one lipophilic or hydrophilic adjuvant chosen from preservatives, antioxidants, fragrances, fillers, screening agents, sequestering agents, essential oils, colouring substances, hydrophilic or lipophilic active agents and lipid vesicles.

14. Composition according to any one of the preceding claims, characterized in that it is provided in the form of an emulsion and in that it additionally comprises at least one dispersant chosen from emulsifiers, vesicles and particles.

15. Cleansing composition for the skin and/or keratinous fibres, containing at least one water-sensitive active agent with a cleansing action and at least one polyol, characterized in that the said composition contains no calcium salt, in that the polyol is present in a quantity which is effective for obtaining a water activity value of the composition of less than or equal to 0.85, and in that the said composition comprises at least one acrylic or methacrylic homopolymer, complexing the water and the polyol(s), and/or an oil.

16. Use, in a composition for topical application which contains no calcium salt and contains at least one water-sensitive active agent with a topical action, of at least one polyol in a quantity which is effective for obtaining a water activity value of the composition of less than or equal to 0.85, and of at least one acrylic or methacrylic homopolymer, complexing the water and the polyol(s), and/or an oil, with the aim of stabilizing the water-sensitive active agent.

17. Cosmetic method of cleansing and/or protecting the skin and/or keratinous fibres, characterized in that it consists in applying to the skin and/or keratinous fibres a composition according to one of Claims 1 to 15.

**Patentansprüche**

1. Stabile Zusammensetzung zur topischen Anwendung, die mindestens einen wasserempfindlichen Wirkstoff zur topischen Einwirkung und mindestens ein Polyol enthält, dadurch gekennzeichnet, daß sie kein Calciumsalz enthält, daß das Polyol in einer Menge vorliegt, die ausreichend ist, um einen Wert der Wasseraktivität der Zusammensetzung von höchstens 0,85 einzustellen, und dadurch, daß sie mindestens ein Acryl- oder Methacrylhomopolymer, das Wasser und das Polyol oder die Polyole komplexiert, und/oder ein Öl enthält.

2. Zusammensetzung nach Anspruch 1, dadurch gekennzeichnet, daß das Polyol in einer Menge vorliegt, die ausreichend ist, um einen Wert der Wasseraktivität der Zusammensetzung von höchstens 0,7 einzustellen.

3. Zusammensetzung nach einem der vorhergehenden Ansprüche, dadurch gekennzeichnet, daß das Polyol in einem Mengenanteil von mindestens 40 Gew.-%, bezogen auf das Gesamtgewicht der Zusammensetzung, vorliegt.

4. Stabile Zusammensetzung zur topischen Anwendung, die mindestens einen wasserempfindlichen Wirkstoff zur topischen Einwirkung und mindestens ein Polyol enthält, dadurch gekennzeichnet, daß das Polyol in einem Mengenanteil im Bereich von 40 bis 99,99 Gew.-%, bezogen auf das Gesamtgewicht der Zusammensetzung, vorliegt, und dadurch, daß sie mindestens ein Acryl- oder Methacrylhomopolymer, das Wasser und das Polyol oder die Polyole komplexiert, und/oder ein Öl enthält.

5. Zusammensetzung nach einem der vorhergehenden Ansprüche, dadurch gekennzeichnet, daß das Polyol unter Glycerin und den Glykolen ausgewählt ist.

6. Zusammensetzung nach einem der vorhergehenden Ansprüche, dadurch gekennzeichnet, daß das Polymer, das Polyol und das gebundene Wasser in einem Mengenanteil von 70 bis 99,99 Gew.-%, bezogen auf das Gesamtgewicht der Zusammensetzung, vorliegen.

7. Zusammensetzung nach einem der vorhergehenden Ansprüche, dadurch gekennzeichnet, daß das Öl in einem Mengenanteil von 5 bis 60 Gew.-%, bezogen auf das Gesamtgewicht der Zusammensetzung, vorliegt.

8. Zusammensetzung nach einem der vorhergehenden Ansprüche, dadurch gekennzeichnet, daß das Öl unter den Mineralölen, pflanzlichen Ölen, tierischen Ölen, synthetischen Ölen, Siliconölen und fluorierten Ölen ausgewählt ist.

9. Zusammensetzung nach einem der vorhergehenden Ansprüche, dadurch gekennzeichnet, daß der wasserempfindliche Wirkstoff zur topischen Anwendung ausgewählt ist unter Enzymen, natürlichen Extrakten, Procyanidololigomeren, Vitaminen, Phosphatderivaten, Glucosederivaten, Harnstoff und Rutin.

10. Zusammensetzung nach dem vorhergehenden Anspruch, dadurch gekennzeichnet, daß der wasserempfindliche Wirkstoff zur topischen Anwendung ausgewählt ist unter einer Protease, grünem Tee, Ascorbinsäure, Retinol und Retinolestern.

11. Zusammensetzung nach einem der vorhergehenden Ansprüche, dadurch gekennzeichnet, daß der wasserempfindliche Wirkstoff in einer Konzentration im Bereich von 0,001 bis 15 Gew.-%, bezogen auf das Gesamtgewicht der Zusammensetzung, vorliegt.

12. Zusammensetzung nach einem der vorhergehenden Ansprüche, dadurch gekennzeichnet, daß sie ferner mindestens ein Salz enthält, das unter den Magnesiumsalzen und den Natriumsalzen ausgewählt ist.

13. Zusammensetzung nach einem der vorhergehenden Ansprüche, dadurch gekennzeichnet, daß sie ferner mindestens einen lipophilen oder hydrophilen Zusatzstoff enthält, der ausgewählt ist unter Konservierungsmitteln, Antioxidantien, Parfums, Füllstoffen, Filtern, Maskierungsmitteln, etherischen Ölen, Färbemitteln, hydrophilen oder lipophilen Wirkstoffen und Lipidvesikeln.

14. Zusammensetzung nach einem der vorhergehenden Ansprüche, dadurch gekennzeichnet, daß sie in Form einer Emulsion vorliegt und ferner mindestens ein Dispergiermittel enthält, das unter den Emulgatoren, Vesikeln und Partikeln ausgewählt ist.

15. Zusammensetzung zur Reinigung der Haut und/oder der Keratinfasern, die mindestens einen wasserempfindlichen, reinigenden Wirkstoff und mindestens ein Polyol enthält, dadurch gekennzeichnet, daß sie kein Calciumsalz enthält, daß das Polyol in einem Mengenanteil vorliegt, der ausreichend ist, um einen Wert der Wasseraktivität der Zusammensetzung von höchstens 0,85 einzustellen, und dadurch, daß sie mindestens ein Acryl- oder Methacrylhomopolymer, das Wasser und das Polyol oder die Polyole komplexiert, und/oder ein Öl enthält.

16. Verwendung mindestens eines Polyols in einem Mengenanteil, der ausreichend ist, um einen Wert der Wasseraktivität der Zusammensetzung von höchstens 0,85 einzustellen, und eines Acryl- oder Methacrylhomopolymers, das Wasser und das Polyol oder die Polyole komplexiert, und/oder eines Öls in einer Zusammensetzung zur topischen Anwendung, die kein Calciumsalz enthält und die mindestens einen wasserempfindlichen Wirkstoff zur topischen Anwendung enthält, um den wasserempfindlichen Wirkstoff zu stabilisieren.

17. Kosmetisches Verfahren zur Reinigung und/oder zum Schutz der Haut und/oder der Keratinfasern, dadurch gekennzeichnet, daß es darin besteht, auf die Haut und/oder die Keratinfasern eine Zusammensetzung nach einem der Ansprüche 1 bis 15 aufzutragen.